# EUROPEAN PATENT APPLICATION

(11) **EP 2 000 158 A1**
(43) Date of publication of application: **10.12.2008**
(21) Application number: 08104233.5
(22) Date of filing: 03.06.2008
(51) Int. Cl.: A61L 27/24, A61L 27/54

(54) **Vascular prosthesis**

(30) Priority: 07.06.2007 CZ 20070396
(71) Applicant: Vyzkumny ustav pletarsky, a.s., 65861 Brno (CZ)
(72) Inventor: Janca, Tomas, 15000 Praha (CZ); Zizkova, Vera, 63500 Brno (CZ); Kizek, Rene, 67921 Cerna Hora (CZ)
(74) Representative: Kratochvil, Vaclav

(57) **Abstract**

The invention relates to vascular prosthesis made of synthetic, optionally textile material combined with collagen including of from 2 to 25 % of weight collagens and from 0.0001 to 1 % of weight adiponectin

## Description

The invention relates to vascular prosthesis made of synthetic material combined with an active substance, optionally proteins are used.

Different solutions of vascular prosthesis are known from technical practice made of various materials, both covered and not covered by a layer of biologically active material. The exemplar vascular prosthesis is described e.g. in patent CZ 277367.

No material used so far for production of vascular prostheses affects unfavourably endotelization of the vascular prosthesis.

The above mentioned deficiencies are mostly eliminated by a vascular prosthesis made of synthetic, optionally textile material combined with proteins, in accordance with this invention. Such vascular prosthesis contains from 2 to 25 % of weight collagen and from 0.0001 to 1 % of weight adiponectin.

Adiponectin may be fixed to a collagen protein or may be in a free bond with the collagen protein.

The vascular prosthesis under this invention is optionally created by a fabric forming a permanent structure, whilst the impenetrability of the prosthesis in case of implantation is secured with collagen. Upon implantation, the collagen layer is gradually absorbed and its absorbancy is extended by hardening.

Polymers and oligomers of adiponectin, both recombination and native, are included in the collagen layer. Adiponectin is a hormone pertaining to the human body produced by adipocytes. Adiponectin increases cellular sensitivity to insulin and increases collection of glucose by such cells. Furthermore, it favourably affects creation of atherosclerotic plates, increases creation of endothelial cells and neoangiogenesis.

Vascular prosthesis with adiponectin under this invention shall be much more rapidly covered upon application by neointima. The released adiponectin will in case of diabetic patients serve for a better and faster cure of the surgical wound.

The exemplar vascular prosthesis made of synthetic textile material combined with collagen contains 10 % of weight collagen and 0.5 % of weight adiponectin. Adiponectin is fixed to collagen protein.

Vascular prosthesis made of synthetic, optionally textile material combined with proteins under this invention may be applied in vascular surgery for reconstruction of arteries for which a vascular prosthesis of the corresponding type is suitable. These types of prostheses may be optionally used in the aortofemoral and ilikofemoral areas and femoropopliteal area over the knee joint, in the area of extracranial arteries and for extraanatomic bypasses.

## Claims

1. Vascular prosthesis of synthetic, optionally textile material combined with collagen *is specific due* to the contents of from 2 to 25 % of weight collagens and from 0.0001 to 1 % of weight adiponectin.

2. Vascular prosthesis under requirement 1 *is specific due* to the fact that adiponectin is fixed to the collagen protein.

3. Vascular prosthesis under requirement 1 *is specific due* the fact that adiponectin is in a free bond with the collagen protein.

4. Vascular prosthesis under requirement 1 *is specific due* the fact that adiponectin is in a free bond with the structure of the prosthesis formed by a synthetic material.
